Europäisches Patentamt

European Patent Office

Office européen des brevets

(11) Veröffentlichungsnummer: **0 315 081 B1**

# EUROPÄISCHE PATENTSCHRIFT

(45) Veröffentlichungstag der Patentschrift: 03.08.94

(21) Anmeldenummer: 88118038.4

(22) Anmeldetag: 29.10.88

(51) Int. Cl.5: **C12N 15/00**, C12P 21/02, C12P 21/00, C07K 13/00, C12N 1/20, A61K 37/02, C12Q 1/68, G01N 33/577

Die Akte enthält technische Angaben, die nach dem Eingang der Anmeldung eingereicht wurden und die nicht in dieser Patentschrift enthalten sind.

(54) Anticoagulatorisches Protein PP4-X, seine Herstellung und Verwendung.

(30) Priorität: 03.11.87 DE 3737237

(43) Veröffentlichungstag der Anmeldung:
10.05.89 Patentblatt 89/19

(45) Bekanntmachung des Hinweises auf die Patenterteilung:
03.08.94 Patentblatt 94/31

(84) Benannte Vertragsstaaten:
AT BE CH DE ES FR GB GR IT LI LU NL SE

(56) Entgegenhaltungen:
WO-A-88/05659

THE EMBO JOURNAL, Band 6, Nr. 6, Juni 1987, Seiten 1599-1604; K. WEBER et al.:"The amino acid sequence of protein II and is its phosphorylation sitefor protein kinase C; the domain structure Ca2+-modulated lipid binding proteins"

(73) Patentinhaber: BEHRINGWERKE Aktiengesellschaft
Postfach 1140
D-35001 Marburg(DE)

(72) Erfinder: Grundmann, Ulrich, Dr.
Am Pfahltor
D-3551 Lahntal-Grossfelden(DE)
Erfinder: Abel, Karl-Josef, Dr.
Am Ziegenberg 6
D-3550 Marburg(DE)
Erfinder: Amann, Egon, Dr.
Sachsenring 8
D-3550 Marburg(DE)

PROC. NATL. ACAD. SCI. USA, Band 85, Juni 1988, Seiten 3708-3712, Washington,DC, US; U. GRUNDMANN et al.: "Characterization of cDNA encoding human placental anticoagulant protein (PP4): Homology with the lipocortin family"

BEHRING INST. MITT., Nr. 82, 1988, Seiten 59-67, Marburg, DE; U. GRUNDMANN etal.: "Isolation and expression of cDNA coding for a new member of thephospholipase A2 inhibitor family"

J. BIOCHEM., VOL. 102, pp. 1261-1273, 1987

EP 0 315 081 B1

**Beschreibung**

Von H. Bohn (Deutsche Offenlegungsschrift DE-A 33 15 000) ist das Protein PP4 aus humanen Plazenten erstmals beschrieben und mittels physikalisch-chemischer Parameter charakterisiert worden. Es wurde nun nach der cDNA für PP4 in einer Placenta-cDNA-Bank mit Hilfe verschiedener, für PP4-Peptide kodierender Oligonukleotide gesucht. Überraschenderweise wurde eine cDNA gefunden, die für ein Protein kodiert, das von PP4 verschieden ist. Daher wird die hier beschriebene cDNA und das resultierende Protein als PP4-X bezeichnet. Die Aminosäuresequenz sowie die Proteinstruktur mit 4 "Repeats" ist den Proteinen Lipocortin I und II ähnlich.

Die Proteine Lipocortin I und II bzw. Calpactin II und I sind seit kurzem als Inhibitoren der Phospholipase A 2 bekannt (F. Davidson et al. (1987), J. Biol. Chem., 1698-1705; Übersicht siehe: M. J. Geisow und J. H. Walker (1986), Trends in Biological Sciences 11, 420-423). Während das Lipocortin I als Substrat für die EGF-Rezeptor-Tyrosin-Proteinkinase fungiert, ist Lipocortin II das dem pp36, isoliert aus embryonalen Hühnerfibroblasten oder intestinalen Epithelzellen von Rindern, als hauptsächlichem Substrat der pp60$^{src}$-Kinase analoge humane Protein (K.-S. Huang et al. (1986) Cell 46, 191-199). Lipocortin II ist zusammen mit Actin Bestandteil des inneren Membrangerüsts bzw. äußeren Cytoskeletts. Lipocortin I besitzt stark entzündungshemmende Eigenschaften analog den Corticosterioden und es wird vermutet, daß die Synthese dieser Proteine durch Corticosteroide induziert wird (G. Cirino et al. (1987) Nature 328, 270-272). Aufgrund von DNA-Sequenzanalysen und partieller Aminosäuresequenzanalysen beider Proteine sind sowohl die Nucleinsäuresequenzen als auch die abgeleiteten Proteinsequenzen bekannt (B. Wallner et al. (1986) Nature 320, 77-81 und K.-S. Huang et al., a.a.O.). Die zwei Sequenzen sind untereinander sehr ähnlich und weisen darüber hinaus jeweils vier Repeatstrukturen auf, die eine "Consensus-Sequenz" von etwa 20 Aminosäuren beinhaltet. Diese vier "Repeats" einer einzigen zugrunde liegenden Aminosäurensequenz sind wahrscheinlich für die - bei Anwesenheit von Ca$^{2+}$ - membranbindenden Eigenschaften der Lipocortine erforderlich.

Obwohl PP4-X von uns mit von PP4 abgeleiteten Oligonucleotidsequenzen gefunden wurde, ist es nicht mit PP4 identisch. Das beruht darauf, daß gerade ein für die Suche verwendetes Oligonukleotid eine Basensequenz enthält, die sowohl in den "Repeats" von Lipocortin I und II als auch denen von PP4 und PP4-X relativ stark konserviert ist.

PP4-X ist ein körpereigenes Antikoagulans, das besonders in stark vaskularisiertem Gewebe wie der Placenta und Gefäßen vorkommt. Durch die antikoagulatorischen Eigenschaften eignet sich PP4-X zum Einsatz als Inhibitor in der Gerinnungskaskade, da es die Blutgerinnung reversibel auf der Stufe des Thromboplastins hemmt. PP4-X bindet über Ca-Ionen an eine negative geladene Phospholipidoberfläche, von der es mit EDTA wieder freigesetzt werden kann. Daraus resultiert eine Anwendung in der Thromboseprophylaxe, weil PP4-X ohne proteolytische Inaktivierung bzw. Aktivierung von Gerinnungsfaktoren bzw. -inhibitoren die Gerinnung selektiv, wirksam und reversibel unterbricht. Das Potential der Gerinnungsfähigkeit bleibt somit voll erhalten.

Zur Gewinnung von partiellen Aminosäuresequenzen und daraus abgeleiteten geeigneten Oligonukleotid-Sonden wurde das Protein PP4 in Bromcyanfragmente zerlegt, von denen zwei Fragmente (41 bzw. 44 Aminosäuren) sequenziert wurden. Folgende Sequenzen wurden ermittelt:

PP4-Oligopeptid-A

MKGLGTDEES ILTLLTSRSN AQRQEISAAF KTLFGRDLLD D

PP4-Oligopeptid-B

MLVVLLQANRD PDAGIDEAQV EQDAQALFQA GELKXGTDEE KFI

Aus dem Oligopeptid A wurde eine Oligonukleotidsequenz mit 35 Basen

ATGAAGGGCC TGGGCACAGA TGAGGAGAGC ATCCT

(PP4-Oligonukleotid 125)

und aus dem Oligopeptid B eine Sequenz mit 36 Basen

GATGCCCAGG CCCTGTTCCA GGCTGGCGAG CTGAAG

(PP4-Oligonukleotid 104)

nach statistischen Daten von R. Lathe (J. Mol. Biol. (1985), 183, 1-12) ausgewählt.

Mit diesen Oligonukleotidsonden wurde eine cDNA-Bank einem Screening unterworfen, die aus mRNA von reifer, humaner Plazenta hergestellt worden war. Zunächst wurde aus der Plazenta die mRNA isoliert und daraus die cDNA hergestellt. Diese wurde mit EcoRI-Enden versehen und die EcoRI-Schnittstelle des Phagenvektors λgt10 ligiert. 46 Klone, die mit den obengenannten Sonden identifiziert worden waren, wurden weiter analysiert. Die Sequenzierung nach an sich bekannten Methoden ergab die DNA-Sequenz, die für PP4-X codiert.

3

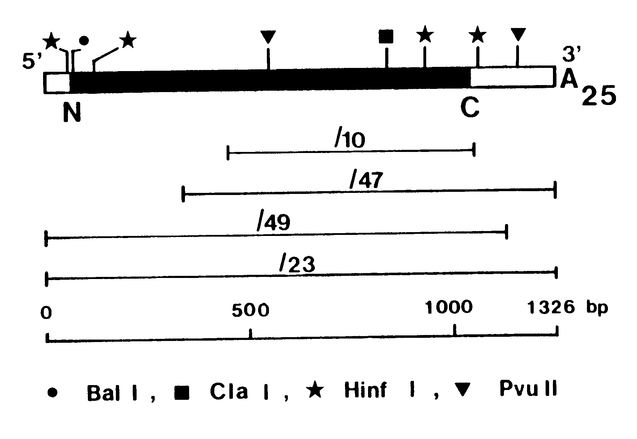

Die Figur zeigt die Restriktionskarte der cDNA-Sequenz, die PP4-X codiert. "N" bezeichnet den N-Terminus, "C" den C-Terminus des kodierenden Bereichs, "A (25)" die Poly-(A)-Sequenz von 25 Basen. Die cDNA-Sequenz repräsentiert die gesamte codierende Sequenz von PP4-X. Die Tabelle 1 zeigt die ermittelte DNA-Sequenz (codierender Strang) und die daraus abgeleitete Aminosäuresequenz eines Klones (PP4-X/23). Die gesamte cDNA hat eine Länge von 1326 Basenpaaren und einen offenen Leserahmen von 972 Basenpaaren. In der Tabelle

```
                          T A B E L L E    1
          10                    30                   50
   CGCAGAGGAGGAGCGCAGCCGGCCTCGAAGAACTTCTGCTTGGGTGGCTGAACTCTGATC

          70                    90                  110
   TTGACCTAGAGTCATGGCCATGGCAACCAAAGGAGGTACTGTCAAAGCTGCTTCAGGATT
                M   A   M   A   T   K   G   G   T   V   K   A   A   S   G   F

         130                   150                  170
   CAATGCCATGGAAGATGCCCAGACCCTGAGGAAGGCCATGAAAGGGCTCGGCACCGATGA
    N   A   M   E   D   A   Q   T   L   R   K   A   M   K   G   L   G   T   D   E

         190                   210                  230
   AGACGCCATTATTAGCGTCCTTGCCTACCGCAACACCGCCCAGCGCCAGGAGATCAGGAC
    D   A   I   I   S   V   L   A   Y   R   N   T   A   Q   R   Q   E   I   R   T

         250                   270                  290
   AGCCTACAAGAGCACCATCGGCAGGGACTTGATAGACGACCTGAAGTCAGAACTGAGTGG
    A   Y   K   S   T   I   G   R   D   L   I   D   D   L   K   S   E   L   S   G

         310                   330                  350
   CAACTTCGAGCAGGTGATTGTGGGGATGATGACGCCCACGGTGCTGTATGACGTGCAAGA
    N   F   E   Q   V   I   V   G   M   M   T   P   T   V   L   Y   D   V   Q   E

         370                   390                  410
   GCTGCAAAGGGCCATGAAGGGAGCCGGCACTGATGAGGGCTGCCTAATTGAGATCCTGGC
    L   Q   R   A   M   K   G   A   G   T   D   E   G   C   L   I   E   I   L   A

         430                   450                  470
   CTCCCGGACCCCTGAGGAGATCCGGCGCATAAGCCAAACCTACCAGCAGCAATATGGACG
    S   R   T   P   E   E   I   R   R   I   S   Q   T   Y   Q   Q   Q   Y   G   R

         490                   510                  530
   GAGCCTTGAAGATGACATTCGCTCTGACACATCGTTCATGTTCCAGCGAGTGCTGGTGTC
    S   L   E   D   D   I   R   S   D   T   S   F   M   F   Q   R   V   L   V   S

         550                   570                  590
   TCTGTCAGCTGGTGGGAGGGATGAAGGAAATTATCTGGACGATGCTCTCGTGAGACAGGA
    L   S   A   G   G   R   D   E   G   N   Y   L   D   D   A   L   V   R   Q   D

         610                   630                  650
   TGCCCAGGACCTGTATGAGGCTGGAGAGAAGAAATGGGGGACAGATGAGGTGAAATTTCT
    A   Q   D   L   Y   E   A   G   E   K   K   W   G   T   D   E   V   K   F   L

         670                   690                  710
   AACTGTTCTCTGTTCCCGGAACCGAAATCACCTGTTGCATGTGTTTGATGAATACAAAAG
    T   V   L   C   S   R   N   R   N   H   L   L   H   V   F   D   E   Y   K   R

         730                   750                  770
   GATATCACAGAAGGATATTGAACAGAGTATTAAATCTGAAACATCTGGTAGCTTTGAAGA
    I   S   Q   K   D   I   E   Q   S   I   K   S   E   T   S   G   S   F   E   D

         790                   810                  830
   TGCTCTGCTGGCTATAGTAAAGTGCATGAGGAACAAATCTGCATATTTTGCTGAAAAGCT
    A   L   L   A   I   V   K   C   M   R   N   K   S   A   Y   F   A   E   K   L

         850                   870                  890
   CTATAAATCGATGAAGGGCTTGGGCACCGATGATAACACCCTCATCAGAGTGATGGTTTC
    Y   K   S   M   K   G   L   G   T   D   D   N   T   L   I   R   V   M   V   S
```

```
          910                    930                    950
TCGAGCAGAAATTGACATGTTGGATATCCGGGCACACTTCAAGAGACTCTATGGAAAGTC
 R   A   E   I   D   M   L   D   I   R   A   H   F   K   R   L   Y   G   K   S

          970                    990                   1010
TCTGTACTCGTTCATCAAGGGTGACACATCTGGAGACTACAGGAAAGTACTGCTTGTTCT
 L   Y   S   F   I   K   G   D   T   S   G   D   Y   R   K   V   L   L   V   L

         1030                   1050                   1070
CTGTGGAGGAGATGATTAAAATAAAAATCCCAGAAGGACAGGAGGATTCTCAACACTTTG
 C   G   G   D   D

         1090                   1110                   1130
AATTTTTTTAACTTCATTTTTCTACACTGCTATTATCATTATCTCAGAATGCTTATTTCC

         1150                   1170                   1190
AATTAAAACGCCTACAGCTGCCTCCTAGAATATAGACTGTCTGTATTATTATTCACCTAT

         1210                   1230                   1250
AATTAGTCATTATGATGCTTTAAAGCTGTACTTGCATTTCAAAGCTTATAAGATATAAAT

         1270                   1290                   1310
GGAGATTTTAAAGTAGAAATAAATATGTATTCCATGTTTTTAAAAAAAAAAAAAAAAAAA
```

AAAAAA

1 sind die Positionen der Oligonukleotidsonden 104 und 125 markiert, die zwischen den Positionen 599 und 634 bzw. 851 und 885 hybridisieren.

Erfindungsgemäß kann die kodierende cDNA mittels geeigneter Expressionssysteme dazu benutzt werden, PP4-X zu exprimieren. Durch die Auswahl des Wirtes kann weiterhin die Form der Modifikation von PP4-X beeinflußt werden. So findet in Bakterien keine, in Hefezellen eine andere Glykosylierung als in höheren eukaryotischen Zellen statt.

In Kenntnis der Aminosäuresequenz von PP4-X ist es möglich, nach konventionellen oder gentechnischen Methoden Aminosäure-Teilsequenzen herzustellen, die als Antigene zur Herstellung von polyklonalen oder monoklonalen Antikörpern dienen können. Solche Antikörper können nicht nur zu diagnostischen Zwecken, sondern auch zur Herstellung von Antikörpersäulen dienen, mit denen PP4-X aus Lösungen abgetrennt werden kann, die es neben anderen Proteinen enthalten.

Mit Hilfe der cDNA bzw. Teilen davon kann man auch auf einfache Weise aus einer genomischen Bank den für PP4-X codierenden genomischen Klon isolieren, mit dessen Hilfe nicht nur eine Expression in eukaryotischen Zellen erleichtert wird, sondern auch weitere diagnostische Aussagen getroffen werden können.

Die Erfindung ist ferner in den Patentansprüchen definiert und in folgenden Beispielen weiter ausgeführt.

Soweit nicht im Text erläutert, werden die folgenden Abkürzungen verwendet:
EDTA = Natrium-ethylendiamin-tetraacetat
SDS = Natrium-dodecylsulfat
DTT = Dithiothreitol
BSA = Rinderserumalbumin

**Beispiele:**

1. Isolierung von RNA aus humaner Plazenta

RNA wurde aus reifer, humaner Plazenta (nach Chirgwin et al., Biochemistry 18 (1979) 5294-5299) gewonnen. Etwa 10 g Plazentagewebe wurden in flüssigem Stickstoff zermörsert, in 80 ml 4 M Guanidinium-Thiocyanat mit 0,1 M Mercaptoethanol suspendiert und 90 sec. bei 20 000 rpm in einem Homogenisator (Ultraturrax) behandelt. Das Lysat wurde 15 min. bei 7 000 rpm zentrifugiert (Sorvall-GSA Rotor) und der Überstand mit 2 ml 1 M Essigsäure und 60 ml Ethanol abs. bei -20°C über Nacht gefällt. Nach Sedimentation bei 6 000 rpm und -10°C für 10 min. wurden die Nucleinsäuren in 40 ml 7,5 M Guanidinium-Hydrochlorid (pH 7,0) vollständig gelöst und mit einer Mischung aus 1 ml 1 M Essigsäure und 20 ml Ethanol abs. gefällt. Zur Abtrennung der DNA wurde die Fällung ein weiteres Mal mit jeweils halben Volumina wiederholt. Die RNA wurde in 12 ml $H_2O$ gelöst, mit einer Mischung aus 1,2 ml 4 M Kaliumacetat und 24 ml Ethanol abs. gefällt, sedimentiert und schließlich erneut in 10 ml $H_2O$ (1 ml pro g Gewebe) aufgenommen.

2. Gewinnung von Poly(A)-haltiger Plazenta-mRNA

Die Plazenta-RNA wurde zur Gewinnung von Poly(A)-haltiger mRNA über Oligo(dT)-Cellulose-Chromatographie (Aviv and Leder, Proc. Natl. Acad. Sci. USA 69 (1973) 1408-1412) in 2 ml Pasteurpipetten in LiCl aufgetrennt. Etwa 5 mg Plazenta-RNA wurden in Puffer 1 (500 mM LiCl, 20 mM Tris (pH 7,5), 1 mM EDTA, 0,1 % SDS) auf die Säule aufgetragen. Während die Poly(A)$^+$-RNA an Oligo(dT)-Cellulose gebunden wurde, konnte die Poly(A)$^-$-RNA wieder eluiert werden. Nach einem Waschschritt mit Puffer 2 (100 mM LiCl, 29 mM Tris (pH 7,5), 1 mM EDTA, 0,1 % SDS) wurde die Poly(A)$^+$-RNA (Plazenta-mRNA) mit Puffer 3 (5 mM Tris (pH 7,5), 1 mM EDTA, 0,05 % SDS) von der Säule eluiert.

Zur weiteren Reinigung wurde die Poly(A)$^+$-RNA auf Puffer 1 eingestellt und erneut über Oligo(dT)-Cellulose chromatographiert.

Die Ausbeute an Plazenta Poly(A)$^+$-RNA betrug nach diesem zweiten Reinigungsschritt etwa 4 % der eingesetzten RNA.

3. Synthese von cDNA aus humaner Plazenta (Plazenta-cDNA) und doppelsträngiger cDNA (dsDNA)

Poly(A)-haltige Plazenta-mRNA wurde vor der cDNA-Synthese im 1,5 % Agarosegel auf Intaktheit geprüft.

Danach wurden 4 μg Plazenta-mRNA in 65,5 μl $H_2O$ gelöst, 10 min. bei 70°C denaturiert und im Eis wieder abgekühlt. Die cDNA-Synthese erfolgte in einem 100 μl-Ansatz nach Zugabe von 20 μl RT$_1$-Puffer (250 mM Tris (pH 8,2) bei 42°C, 250 mM KCl, 30 mM MgCl$_2$), 2,5 μl 20 mM dNTP (d.h. aller vier Desoxynukleosidtriphosphate), 1 μl Oligo(dT) von 1 μg/ml, 1 μl 1 M DTT, 2 μl RNAsin und 8 μl Reverse Transcriptase (24 U/μl) für 90 min. bei 42°C. Doppelsträngige cDNA (dsDNA) wurde nach Gubler and Hoffmann (Gene 25 (1983) 263-269) synthetisiert. Die Synthese erfolgte unmittelbar nach der cDNA-Synthese durch Zugabe von 305,5 μl $H_2O$, 80 μl RT$_2$-Puffer (100 mM Tris (pH 7,5), 25 mM MgCl$_2$, 500 mM KCl, 50 mM DTT, 250 μg/ml BSA), 2 μl RNase H (2 U/μl), 2,5 μl E. Coli DNA Ligase (5 U/μl), 5 μl 15 mM β-NAD, und 5 μl DNA Polymerase I (5 U/μl) und Inkubation für 5 h bei 15°C. Durch Hitzeinaktivierung (70°C, 30 min.) wurde die Reaktion beendet.

Der Reaktionsansatz wurde nach Zugabe von 55 μl 250 μM dNTP, 55 μl 10 mM Tris (pH 7,5), 10 mM MgCl$_2$, 10 μg/ml BSA, 3 μl T4 DNA-Polymerase I (1 U/μl), 2 μl RNase H (2 U/μl) und 2 μl RNase A (2 μg/ml) für weitere 30 min. bei 37°C inkubiert, um die Vollständigkeit der Synthese am zweiten DNA-Strang zu gewährleisten ("Repair Reaction").

4. Ligierung von EcoRI-Linkern an die dsDNA und Öffnen der Linker.

Zur Errichtung einer Plazenta-cDNA-Bank wurde die dsDNA mit EcoRI-Enden versehen, um sie in die EcoRI-Schnittstelle des Phagenvektors λgt10 ligieren zu können (T. Maniatis et al. (1982), Molecular Cloning, A Laboratory Manual, Cold Spring Harbor). Hierzu wurde die dsDNA
a) mit EcoRI-Methylase behandelt, um interne EcoRI-Schnittstellen der dsDNA zu schützen,
b) mit EcoRI-Linkern versehen, die
c) danach mit EcoRI geöffnet wurden.

Zu a):

Die Methylase-Reaktion der dsDNA erfolgte direkt im Anschluß an die "Repair Reaction" nach Zugabe von 25 μl 500 mM EDTA (pH 8,0), 60 μl Methylase-Puffer (100 mM NaOAc (pH 5,2), 2 mg S-Adenosyl-L-Methionin) und 2 μl EcoRI-Methylase (20 U/μl) durch Inkubation bei 37°C für 30 min.

Der Reaktionsansatz wurde mit Phenol extrahiert und die dsDNA mit 60 μl 4M NaOAc und 1300 μl Ethanol gefällt. Die dsDNA wurde zweimal mit 70 % Ethanol gewaschen, einmal mit Ether ausgeschüttelt und getrocknet.

Zu b):

Die EcoRI-methylierte dsDNA wurde in 88 $\mu$l $H_2O$ gelöst und nach Zugabe von 10 $\mu$l Ligase-Puffer (500 mM Tris (pH 7,4), 100 mM $MgCl_2$, 100 mM DTT, 10 mM Spermidin, 10 mM ATP, 1 mg/ml BSA), 1 $\mu$l T4 DNA-Ligase (10 U/$\mu$l) mit 1 $\mu$l EcoRI Linkern (0,5 $\mu$g/$\mu$l) (pGGAATTCC bzw. pAGAATTCT) über Nacht bei 15°C ligiert.

Zu c):

Das Volumen der Ligase-Ansatzes wurde mit 6 $\mu$l $H_2O$, 12 $\mu$l 10x EcoRI-Puffer und 2 $\mu$l EcoRI (120 U/$\mu$l) auf 120 $\mu$l gebracht. Die EcoRI-Verdauung erfolgte für 2 h bei 37°C.

5. Abtrennen nichtgebundener Linker über Kaliumacetat-Gradienten und Größenselektionierung der dsDNA

Zur Abtrennung aller nichtgebundenen EcoRI-Linker von der dsDNA wurde der EcoRI-Reaktionsansatz in toto auf einen Kaliumacetat-Gradienten (5-20 % KOAc, 1 mM EDTA, 1 $\mu$l/ml Ethidiumbromid) aufgetragen und 3 h bei 50 000 rpm und 20°C zentrifugiert (Beckman SW 65-Rotor).
Der Gradient wurde von unten so fraktioniert, daß die ersten fünf Fraktionen 500 $\mu$l maßen und alle restlichen 100 $\mu$l. Die Fraktionen wurden mit 0,01 Volumen Acrylamid ( 2 mg/ml) und 2,5 Volumen Ethanol gefällt, einmal mit 70 %igem Ethanol gewaschen, getrocknet und jeweils in 5 $\mu$l $H_2O$ auf genommen.
Zur Größenbestimmung der dsDNA wurden 1 $\mu$l jeder Fraktion im 1,5 % Agarose-Gel analysiert. Zusätzlich wurde mit 1 $\mu$l jeder Fraktion die Quantität an dsDNA bestimmt.
Fraktionen mit dsDNA über 1000 bp wurden vereinigt und die Probe so eingeengt, daß die Endkonzentration 27 $\mu$g/ml betrug.

6. Einbau der dsDNA in den Phagenvektor λgt10 und "in vitro packaging"-Reaktion.

Der Einbau der dsDNA in die EcoRI-Schnittstelle des Phagenvektors λgt10 (Vector Cloning systems, San Diego, CA) erfolgte in einem Ligaseansatz von 4 $\mu$l: 2 $\mu$l dsDNA, 1 $\mu$l λgt10 x EcoRI (1 $\mu$g/ml), 0,4 $\mu$l Ligase-Puffer, 0,5 $\mu$l $H_2O$, 0,1 $\mu$l T4 DNA-Ligase. Der Ansatz wurde 4 h bei 15°C inkubiert.
Zur Etablierung der Plazenta-cDNA-Bank im Phagenvektor λgt10 folgte mit den λ-lysogenen Zellextrakten E. coli NS 428 und NS 433 eine "in vitro packaging"-Reaktion des Ligaseansatzes bei Raumtemperatur für 2 h (Vector Cloning systems, San Diego, CA; Enquist and Sternberg, Methods in Enzymology 68, (1979), 281-298). Die Reaktion wurde mit 500 $\mu$l Suspensionsmedium (SM: 0,1 M NaCl, 8 mM $MgSO_4$, 50 mM Tris (pH 7,5), 0,01 % Gelatine) und 2 Tropfen Chloroform gestoppt.

7. Titerbestimmung und Analyse der Plazenta-cDNA-Bank

Die Zahl der "Plaque Forming Units" (PFU) der Plazenta-cDNA-Bank wurde mit kompetenten Zellen des E. coli-K 12-Stammes C600 HFL bestimmt: Sie betrug 1 x $10^6$ PFU. Etwa 80 % aller Phagen enthielten DNA-Inserts, die größer als 1000 Basenpaare waren.

8. Oligonukleotidsonden zum "Screening" der Plazenta-cDNA-Bank

Zwei Oligonukleotidsonden wurden zur Analyse der Plazenta-cDNA-Bank synthetisiert. Ihre Sequenzen wurden abgeleitet von der Aminosäuresequenz mehrerer Bromcyanfragmente des antikoagulatorischen Proteins PP4.
Die Art des Aufbaus und die Verwendung beider Sonden folgten im wesentlichen den Regeln von R. Lathe, a.a.O..
Beide Oligonukleotidsequenzen wurden mit T4 Polynukleotidkinase unter Anwesenheit von ($\gamma$-32P)ATP am 5′-Ende markiert (ca. 1 $\mu$g DNA ($\gamma$-32P)ATP:3000Ci/mmol,10 $\mu$Ci/$\mu$l, eingesetzt wurden 6 $\mu$l/40 $\mu$l Reaktionsansatz). Die Sonden hatten eine spezifische Aktivität von 1 x 10 8 Bq/$\mu$l bzw. 1,5 x 10 6 Bq/pMol.

9. "Screening" der Plazenta-cDNA mit PP4-spezifischen Oligonukleotiden

1 x $10^6$ PFU der Plazenta-cDNA-Bank wurden mit den PP4-Oligonukleotidsonden 104 und 125 zusammen untersucht. Dazu wurden 3 x $10^4$ PFU mit Zellen des E. coli K 12-Stammes C 600 HFL in Weich-Agar auf 13,5 cm Petrischalen ausplattiert und 6 h bei 37°C inkubiert. Zu diesem Zeitpunkt war noch keine vollständige Lyse eingetreten. Die Platten wurden über Nacht im Kühlschrank inkubiert und die Phagen auf Nitrocellulosefilter (Schleicher & Schüll, BA 85, Ref.-Nr. 401124) übertragen (Duplikate). Nitrocellulosefilter und Petrischalen wurden mit einer Injektionskanüle markiert, um eine spätere Zuordnung zu ermöglichen. Die Petrischalen wurden während des Prozessierens der Nitrocellulosefilter im Kühlraum gelagert. Die auf den Nitrocellulosefiltern befindliche DNA wurde denaturiert, indem die Filter für 5 min. auf mit 1,5 M NaCl, 0,5 M NaOH-getränktes Filterpapier (Whatman-M 3) gelegt wurden. Anschließend wurden die Filter auf die gleiche Art mit 1,5 M NaCl, 0,5 M Tris (pH 8,0) renaturiert und mit 2 x SSPE (0,36 M NaCl, 16 mM NaOH, 20 mM NaH$_2$PO$_4$, 2 mM EDTA) gewaschen. Die Filter wurden dann für 2 h bei 80°C im Vakuum getrocknet. Die Filter wurden für 4 h bei 65°C in 3xSSC, 0,1 % SDS (20 x SSC = 3 M NaCl, 0,3 M Na-Citrat) gewaschen und für 4 h bei 65°C vorhybridisiert (Prähybridisierungslösung: 0,6 M NaCl, 0,06 M Tris (pH 8,3), 6 mM EDTA, 0,2 % nichtionisches synthetisches Saccharose-Polymer ($^R$Ficoll), 0,2 % Polyvinylpyrrolidon 40, 0,2 % BSA, 0,1 % SDS, 50 µg/ml denaturierte Heringssperma-DNA). Die Filter wurden über Nacht unter Zusatz von 100 000-200 000 Bq des markierten Oligonukleotids/ml Hybridisierungslösung (wie Prähybridisierungslösung, jedoch ohne Heringssperma-DNA) in Bechergläsern bzw. in zugeschweißten Polyethylenfolien unter leichtem Schütteln inkubiert. Die Hybridisierungstemperatur betrug 44°C. Die Nitrocellulosefilter wurden mit 6 x SSC, 0,05 M Natriumpyrophosphat eine Stunde bei Raumtemperatur und für eine weitere Stunde bei der jeweiligen Hybridisierungstemperatur gewaschen. Die Filter wurden getrocknet und über Nacht autoradiographiert. Signale auf dem Röntgenfilm, die auf beiden Duplikaten auftraten, wurden der Petrischale zugeordnet und die Region (ca. 50 Plaques) mit dem breiten Ende einer Pasteurpipette ausgestanzt und die Phagen in 1 ml SM-Puffer resuspendiert. Positive Phagen wurden über drei Runden vereinzelt, bis ein einzelner Klon vorlag.

Insgesamt wurden 1 x 10$^6$ PFU der Plazenta-cDNA-Bank untersucht. Es wurden 54 Signale auf Duplikat-Filtern identifiziert. Beim weiteren Screening mit den einzelnen Sonden zeigte sich, daß neuen Klone mit beiden Sonden reagierten. Alle diese neun Klone tragen eine PP4-X kodierende Sequenz mit einer maximalen Länge von 1326 Basenpaaren für den Klon PP4-X/23. Die Sequenzanalyse der PP4-X-Klone zeigte im folgenden, daß über die gesamte Länge beider Sonden je acht "Mismatches" zur ermittelten PP4-X-Sequenz auftreten (Tabelle 2).

## Tabelle 2

### PP4-X. Seq. versus PP4-Oligonukleotid 104

```
596 CAGGATGCCCAGGACCTGTATGAGGCTGGAGAGAAGAAATGGGGGACAGA 645
        ||||||||||| |||||   |||||||| ||| |||
  1 .. GATGCCCAGGCCCTGTTCCAGGCTGGCGAGCTGAAG                36
```

### Tabelle 2, Forts.

### PP4-X. Seq. versus PP4-Oligonukleotid 125

```
846 AATCGATGAAGGGCTTGGGCACCGATGATAACACCCTCATCAGAGTGATG 895
       |||||||| ||||||| |||||  | | | ||| |
  1 ... ATGAAGGGCCTGGGCACAGATGAGGAGAGCATCCT                35
```

## 10. DNA-Sequenzanalyse

Die Phagenklone PP4-X/10, /23, /47 und /49 wurden vermehrt und ihre DNA jeweils extrahiert. Das jeweilige EcoRI-Fragment wurde isoliert und in die EcoRI-Stellen des Vektors pICl9H für Restriktionsanaly-

sen bzw. des Vektors M13mp8 für die Sequenzanalysen mittels der enzymatischen Didesoxymethode nach Sanger einligiert. Die Sequenz zeigt einen offenen Leserahmen und kodiert für ein Protein mit maximal 321 Aminosäuren (Tab. 1).

11. Expression von PP4-X in E.coli

a) Expression des reifen, unfusionierten PP4-X Proteins:

Der Expressionsvector pTrc97A (Europäische Patentanmeldung EP 0 236 978) wurde mit EcoRI verdaut und die überhängenden 5′ Enden mit dem Enzym Mung Bean Nuklease entfernt. Diese Manipulation erzeugte die Sequenz: 5′ AACAGACCATGG 3′. Die DNA wurde dann mit HindIII verdaut und das 2889 Basenpaar große Fragment isoliert. Dieses Fragment wurde mit dem 1165 Basenpaar großen Ball/HindIII Fragment der PP4-X cDNA ligiert, wobei das "glatte", durch Ball produzierte Ende der cDNA mit dem durch EcoRI geschnittenen und Mung Bean Nuklease behandelten Ende des Vektors reagierte. Die resultierende Sequenz, kodierend für den N-Terminus des PP4-X Proteins war:

```
          1    2    3    4    5    6    7

         Met  Ala  Met  Ala  Thr  Lys  Gly

  5' ...AACAGACC ATG  GCC  ATG  GCA  ACC  AAA  GGA ... 3'
```

Das resultierende, 4054 Basenpaare große Expressionsplasmid pPP4-X ist somit in der Lage, die Synthese des reifen, unfusionierten PP4-X Proteins in E. coli zu vermitteln.

Zur Expression des PP4-X Proteins wurde die pPP4-X Plasmid DNA nach bekannten Methoden in den E.coli K12 Stamm W3110LacIQ transformiert, wobei Ampicillin-resistente Kolonien das erwartete PP4-X Protein nach Induktion exprimieren. Analyse der zellulären Expressionsprodukte in der Coomassieblau-gefärbten SDS-PAGE zeigte das Auftreten einer neuen Bande an einer Position, die einem Molekulargewicht von ca. 36 kD entspricht. Analyse der Proteinextrakte von Kontrollzellen, die nicht mit pPP4-X Plasmid DNA transformiert sind, zeigen an entsprechender Stelle diese Proteinbande nicht. Das in den W3110lacIQ (pPP4-X)-Extrakten auftretenden Protein von ca. 36 kD reagiert im Westernblot-Experiment spezifisch mit Anti-PP4-Antiseren. In einem weiteren Experiment wurden die zellulären Proteine der Kultur nach IPTG-Induktion mit 35S-Methionin markiert und zelluläres Protein in der SDS-PAGE mit nachfolgender Autoradiographie analysiert. Auch hier zeigte sich eine prominente Proteinbande entsprechend einem Molekulargewicht von ca. 36 kD. Dieses Protein läßt sich spezifisch mit Anti-PP4-X-Antiseren immunfällen.

b) Expression von PP4-X Fusionsproteinen

In einem weiteren Experiment ein PP4-X Fusionsprotein mit der E.coli β-Galaktosidase hergestellt. Dazu wurde das Plasmid pBD2IC20H (Europäische Patentanmeldung EP 0 236 978 ) mit SmaI und HindIII verdaut und das ca. 3,8 kb große Fragment isoliert und mit dem oben bereits erwähnten Ball/ HindIII Fragment der PP4-X cDNA ligiert. Das resultierende Plasmid pBD2IC20H-PP4-X exprimiert in E.coli ein ca. 77 kD großes Fusionsprotein, welches aus dem ca. 41 kD großen ß-Galaktosidaseanteil und aus dem ca. 36 kD großen PP4-X Anteil besteht. Das Protein wird hoch exprimiert und ist ebenfalls immunreaktiv mit Anti-PP4-X-Antiseren.

Weiterhin wurde ein PP4-X Fusionsprotein mit der DNA-Polymerase des Bakteriophagen MS2 hergestellt.

Dazu wurde das Plasmid pEx31c (K. Strebel et al. (1986), J. Virol. 57, 983-991) mit EcoRI verdaut und die DNA mit Klenow-Polymerase doppelsträngig gemacht. Die DNA wurde dann mit HindIII verdaut und das ca. 3,2 kb große Fragment isoliert und mit dem bereits oben erwähnten Ball/HindIII Fragment der PP4-X cDNA ligiert. Das resultierende Plasmid pEx31c-PP4-X exprimiert unter der Kontrolle des hitzeinduzierbaren Lambda PL Promoters in E.coli ein ca. 46 kD großes Fusionsprotein, welches aus einem ca. 10 kD großen MS2-Polymerase Anteil und dem 36 kD großen PP4-X Anteil besteht. Das Protein wird nach Hitzeinduktion hoch exprimiert und reagiert ebenfalls spezifisch mit Anti-PP4-X-Antiseren.

Die beiden PP4-X-Fusionsproteine stellen diagnostisch geeignete Mittel dar zur Antikörpererzeugung und zum Antikörpernachweis.

EP 0 315 081 B1

**Patentansprüche**

1. PP4-X, gekennzeichnet durch die Aminosäuresequenz gemäß Tabelle 1.

2. DNA-Sequenz, kodierend für PP4-X, enthaltend den kodierenden Strang gemäß Tabelle 1.

3. DNA, die mit der DNA gemäß Anspruch 2 unter stringenten Bedingungen hybridisiert, vorausgesetzt, daß diese DNA für ein Protein mit PP4-X-Aktivität, gemäß Anspruch 1, Kodiert.

4. DNA-Sequenz, kodierend für die Aminosäuresequenz gemäß Tabelle 1.

5. Genstruktur, enthaltend eine DNA gemäß Anspruch 2, 3 oder 4.

6. Vektor, enthaltend eine DNA-Sequenz nach einem oder mehreren der Ansprüche 2 bis 5.

7. Transformierte Zelle, enthaltend DNA nach Anspruch 2, 3, 4 oder 5.

8. PP4-X, erhalten durch Expression einer DNA-Sequenz nach Anspruch 2, 4 oder 5 in Bakterien, Hefen oder tierischen Zellen.

9. Verfahren zur Herstellung von PP4-X, dadurch gekennzeichnt, daß man eine cDNA nach Anspruch 2, 4, 5 oder 6 in ein Expressionssystem einbringt und dort zur Expression bringt.

10. Protein mit PP4-X-Aktivität, enthaltend die Aminossäuresequenz gemäß Tabelle 1 oder eine PP4-X-eigene Teilsequenz davon sowie Varianten dieser Proteine, welche mindestens eine PP4-X- eigene Teilsequenz enthalten.

11. Monoklonale oder polyklonale Antikörper, welche aus dem Protein gemäß Anspruch 10 oder antigenwirksamen Teilen davon erhalten werden und welche an eine PP4-X-eigene Teilsequenz binden.

12. Diagnostikum, enthaltend ein Antigen nach Anspruch 1, 8 oder 10.

13. Diagnostikum, enthaltend Antikörper nach Anspruch 11.

14. Diagnostikum, das eine DNA nach Anspruch 2, 3, 4 oder 5 ganz oder teilweise enthält.

15. Diagnostikum, das eine RNA ganz oder teilweise enthält, die zu der DNA nach Anspruch 2 komplementär ist.

16. Arzneimittel, gekennzeichnet durch einen Gehalt an einem Protein gemäß Anspruch 1, 8 oder 10.

**Claims**

1. PP4-X, which has the amino acid sequence shown in Table 1.

2. A DNA sequence coding for PP4-X and containing the coding strand shown in Table 1.

3. A DNA which hybridizes with the DNA as claimed in claim 2 under stringent conditions, with the proviso that this DNA codes for a protein having PP4-X activity, as in claim 1.

4. A DNA sequence coding for the amino acid sequence shown in Table 1.

5. A gene structure containing a DNA as claimed in claim 2, 3 or 4.

6. A vector containing a DNA sequence as claimed in one or more of claims 2 to 5.

7. A transformed cell containing DNA as claimed in claim 2, 3, 4 or 5.

10

8. PP4-X obtained by expression of a DNA sequence as claimed in claim 2, 4 or 5 in bacteria, yeasts or animal cells.

9. A process for the preparation of PP4-X, which comprises insertion of a cDNA as claimed in claim 2, 4, 5 or 6 into an expression system, and bringing about its expression therein.

10. A protein having PP4-X activity and containing the amino acid sequence as shown in Table 1 or a partial sequence thereof which is intrinsic to PP4-X, as well as variants of these proteins which contain at least one partial sequence which is intrinsic to PP4-X.

11. Monoclonal or polyclonal antibodies which are obtained from a protein as claimed in claim 10 or parts thereof having antigenic activity and which binds to a partial sequence which is intrinsic to PP4-X.

12. A diagnostic aid containing an antigen as claimed in claim 1, 8 or 10.

13. A diagnostic aid containing antibodies as claimed in claim 11.

14. A diagnostic aid which contains a DNA as claimed in claim 2, 3, 4 or 5, in whole or in part.

15. A diagnostic aid which contains an RNA, in whole or in part, which is complementary with the DNA as claimed in claim 2.

16. A pharmaceutical which contains a protein as claimed in claim 1, 8 or 10.

**Revendications**

1. PP4-X, caractérisée par la séquence d'aminoacides selon le tableau 1.

2. Séquence d'ADN codant pour PP4-X, contenant le brin codant selon le tableau 1.

3. ADN qui s'hybride, dans des conditions stringentes, avec l'ADN selon la revendication 2, étant entendu que cet ADN code pour une protéine à activité de PP4-X selon la revendication 1.

4. Séquence d'ADN codant pour la séquence d'aminoacides selon le tableau 1.

5. Structure génique, contenant un ADN selon la revendication 2, 3 ou 4.

6. Vecteur contenant une séquence d'ADN selon une ou plusieurs des revendications 2 à 5.

7. Cellule transformée, contenant de l'ADN selon la revendication 2, 3, 4 ou 5.

8. PP4-X, obtenue par expression d'une séquence d'ADN selon la revendication 2, 4 ou 5, dans des bactéries, des levures ou des cellules animales.

9. Procédé pour la production de PP4-X, caractérisé en ce que l'on introduit dans un système d'expression un ADNc selon la revendication 2, 4, 5 ou 6, et on l'y amène à l'expression.

10. Protéine à activité de PP4-X, contenant la séquence d'aminoacides selon le tableau 1 ou une séquence partielle de celle-ci, propre à PP4-X, ainsi que des variantes de ces protéines, qui contiennent au moins une séquence partielle propre à PP4-X.

11. Anticorps monoclonaux ou polyclonaux, qui sont obtenus à partir de la protéine selon la revendication 10 ou de fragments à activité antigénique de celle-ci, et qui forment une séquence partielle propre à PP4-X.

12. Agent de diagnostic contenant un antigène selon la revendication 1, 8 ou 10.

13. Agent de diagnostic contenant des anticorps selon la revendication 11.

**14.** Agent de diagnostic qui contient en partie ou en totalité un ADN selon la revendication 2, 3, 4 ou 5.

**15.** Agent de diagnostic qui contient en partie ou en totalité un ARN qui est complémentaire de l'ADN selon la revendication 2.

**16.** Médicament, caractérisé par une teneur en une protéine selon la revendication 1, 8 ou 10.

5'
N
C
3'
A₂₅

/10
/47
/49
/23

| 0 | 500 | 1000 | 1326 bp |

● Bal I , ■ Cla I , ★ Hinf I , ▼ Pvu II

EP 0 315 081 B1